(19) Europäisches Patentamt

European Patent Office

Office européen des brevets

(11) **EP 0 845 999 B1**

(12) **EUROPEAN PATENT SPECIFICATION**

(45) Date of publication and mention
of the grant of the patent:
**06.03.2002  Bulletin 2002/10**

(51) Int Cl.⁷: **A61K 47/26**, A61K 35/66,
A61K 9/20

(21) Application number: **96929617.7**

(22) Date of filing: **23.08.1996**

(86) International application number:
**PCT/SE96/01043**

(87) International publication number:
**WO 97/07822 (06.03.1997 Gazette 1997/11)**

(54) **METHOD FOR THE PRODUCTION OF TABLETS BY PRESSING AND TABLETS PRODUCED BY THE METHOD**

VERFAHREN ZUR HERSTELLUNG VON TABLETTEN DURCH VERPRESSUNG UND DURCH DIESES VERFAHREN HERGESTELLTE TABLETTEN

PROCEDE DE FABRICATION DE CACHETS PAR PRESSAGE ET CACHETS AINSI OBTENUS

(84) Designated Contracting States:
**AT BE CH DE DK ES FR GB IT LI NL**

(30) Priority:  **25.08.1995  SE 9502941**

(43) Date of publication of application:
**10.06.1998  Bulletin 1998/24**

(73) Proprietor: **Wasa Medicals AB
400 15 Göteborg (SE)**

(72) Inventor: **CEDGARD, Lennart
S-413 02 Göteborg (SE)**

(74) Representative: **Bergquist, Kjell Gunnar et al
Albihns Göteborg AB, Box 142
401 22 Göteborg (SE)**

(56) References cited:
**EP-A- 0 227 441**

• **STN INTERNATIONAL, File CAPLUS, CAPLUS
Accession No. 1992:241966, ASAHI
BREWERIES, LTD., "Lactobacillus-Containing
Tablets Coated with Intestinally Soluble
Substances"; & JP,A,04 041 434 (12-02-92)
Heisei.**

EP 0 845 999 B1

## Description

TECHNICAL FIELD:

The present invention relates to a method for the production of tablets by pressing of tablet material which contains microorganisms.

PRIOR ART:

Tablets are usually produced by pressing of a pulverulent tablet mass in a suitable shape in a so-called tablet punching machine. The tablets may have different shape and be of different size and they may also be of different hardness dependent on the properties of the tablet mass and the pressure to which they are subjected during the punching of the tablets.

When the tablets are formed heat is developed as a result of the friction against the mould surfaces and the inner friction in the tablet mass. Since the tablets usually consist of chemicals and the temperature increase is not too high, this will not create any problem since the chemicals can resist this heat increase and also are cooled rapidly. However, some tablet masses contain living microorganisms, such as bacteria, which are sensitive to high temperatures and because of this some of these bacteria die during the tablet punching.

TECHNICAL PROBLEM:

Tablets which contain microorganisms, for instance in the form of bacteria, and which are intended to contain such organisms will lose a part of or all of their value when the microorganisms are destroyed during the tablet punching. This cannot be avoided by simply using a lower pressure on the conventional tablet mass and thereby creating a lower heat development since the tablet must be subjected to a certain pressure so that it maintains its shape and is not crumbled. For known tablet masses it is not unusual that a reduction of the viability (survival) of the bacteria in the tablet is up to 80% and even more.

SOLUTION:

It has therefore always been a problem to be able to produce tablets which contain microorganisms in the form of bacteria with a lesser reduction of the viability from tablet mass to a complete tablet and therefore according to the invention a method has been obtained for the production of tablets by pressing of tablet material comprising living organisms, which is characterized in that the tablet material also contains fructose oligosaccharides consisting of more than two monosaccharides.

According to the invention, it is suitable that the oligosaccharides consist of inulin.

According to the invention it is suitable that the oligosaccharides are present in an amount of 40-99.5 % by weight of the tablet material.

The tablet material according to the invention can suitably contain microorganisms consisting of lactic acid producing bacteria.

The invention also comprises tablets produced by the method according to the invention, which tablets contain fructose oligosaccharides and microorganisms whereby the oligosaccharides preferably consist of inulin.

The tablets according to the invention may contain lactic acid producing bacteria as microorganisms and they may also contain other additives such as polysaccharides, for example microcrystalline cellulose and starch, as well as other additives such as calcium diphosphate.

DETAILED DESCRIPTION:

The tablets according to the invention comprise microorganisms, preferably lactic acid producing bacteria cultures known as probiotica, which are intended to normalise or balance bacterial flora being present in the stomach and the intestine of humans or animals, but they may also contain other types of bacteria.

By mixing oligosaccharides, preferably fructose oligosaccharides, in the tablet mass as a so-called supporting substance the tablet punching is facilitated, which makes it possible to punch tablets at a lower pressure and lower heat development at the same time as the hardness of the tablet is maintained. The brittleness of the tablet, the friability, is surprisingly not changed with the tablet mass according to the present invention.

Due to this new composition, the punching pressure for the tablet making maybe reduced by up to 50% compared to conventional tablet punching methods without any reduction of the friability. This friability according to the invention will be 0.3-0.5, which is to be compared with the reference values which are accepted according to GMP (Good Manufacturing Practice) which are within the range of 0.1-1.0. The friability is expressed in percent weight reduction of the tablets when they are rotated 100 revolutions in a standard testing machine.

The amount of oligosaccharides depends on different crystalline qualities but may suitably be 99.5-40 weight percent of the total tablet mass without admixing any other supporting substance. However, if desired, known supporting substances such as calcium diphosphate, microcrystalline cellulose and starch may be added in a suitable small amount. A smaller addition of oligosaccharides can, however, give rise to a smaller difference with regard to the viability compared with tablet masses containing only conventional supporting substances.

The tablets according to the present invention have a lower hardness due to the lower punching pressure when the tablets are formed but an increased viability for the strain of bacteria, which makes every tablet more efficient than conventional tablets. By not pressing the tablets so hard the yield of tablets for a given amount of tablet mass will also increase.

The invention will be described more in detail below by means of two examples, of which Example 1 describes a method according to the present invention and Example 2 describes a method of conventional kind.

Example 1: recipe having an active substance and tablet filling material

| Str. thermophilus & L. bulgaricus | 50% |
| Bifidobacterium animalis | 0.5% |
| L. plantaris | 0.5% |
| Inulin (fructose oligosaccharides) | 49% |
| | 100% |

| Hardness: 2.75 kp | Friability: 0.3 |
| Viability original granulate: | 5E8 cfu/g |
| Viability tablet: | 3E8 cfu/g |
| 40% reduction of cfu (colony forming units) | |

Example 2: recipe having active substance and tablet filling material

| Str. thermophilus & L. bulgaricus | 50% |
| Bifidobacterium animalis | 0.5% |
| L. plantaris | 0.5% |
| Calcium diphosphate | 20% |
| Microcrystalline cellulose | 18% |
| Starch | 11% |
| | 100% |

| Hardness: 5.5 kp | Friability: 0.3% |
| Viability original granulate: | 5E8 cfu/g |
| Viability tablet: | 1E8 cfu/g |
| 80% reduction of cfu (colony forming units) | |

As appears from the above examples, the friability is maintained unchanged with a value of 0.3 whereas the hardness has been decreased to 2.75 kp compared with 5.5 kp for the conventional method. The viability has increased from 1E8 cfu/g to 3E8 cfu/g according to the invention. The reduction of cfu from tablet mass to tablet during the tablet punching became only 40% according to the new method and 80% according to the conventional method.

Accordingly, the new method results in an increased maintained viability after tablet punching of up to 200% compared with conventional tablet fillers. The increased yield results in an appreciably better economy and quality improvement of the above products.

The invention is not limited to the embodiments shown above but can be varied in different ways within the scope of the claims.

Claims

1. Method for the production of tablets having high viability in the tablet by pressing tablet material containing living microorganisms,
characterized in that the tablet material also contains fructose oligosaccharides consisting of more than two monosaccharides.

2. Method according to claim 1
characterized in that the oligosaccharides are present in an amount of 40-99.5 percent by weight of the tablet material.

3. Method according to any of claims 1-2,
characterized in that the oligosaccharides consist of inulin.

4. Method according to any of claims 1-3,
characterized in that the microorganisms consist of lactic acid producing bacteria.

5. Tablets produced according to any of claims 1-4 containing fructose oligosaccharides and microorganisms.

6. Tablets according to claim 5,
characterized in that the oligosaccharides consist of inulin.

7. Tablets according to any of claims 5-6,
characterized in that the microorganisms consist of lactic acid producing bacteria.

8. Tablets according to any of claims 5-7,
characterized in that they also contain polysaccharides such as microcrystalline cellulose and starch as well as other additives such as calcium diphosphate.

Patentansprüche

1. Verfahren zur Herstellung von Tabletten, die eine hohe Überlebensfähigkeit in der Tablette beim Pressen von lebende Mikroorganismen enthaltendem Tablettenmaterial ergeben,
dadurch gekennzeichnet, dass

das Tablettenmaterial auch Fructoseoligosaccharide enthält, die aus mehr als zwei Monosacchariden bestehen.

**2.** Verfahren gemäß Anspruch 1, **dadurch gekennzeichnet, dass** die Oligosaccharide in einer Menge von 40 bis 99,5 Gew.% des Tablettenmaterials vorhanden sind.

**3.** Verfahren gemäß einem der Ansprüche 1 bis 2, **dadurch gekennzeichnet, dass** die Oligosaccharide aus Inulin bestehen.

**4.** Verfahren gemäß einem der Ansprüche 1 bis 3, **dadurch gekennzeichnet, dass** die Mikroorganismen aus Milchsäure produzierenden Bakterien bestehen.

**5.** Tabletten, hergestellt gemäß einem der Ansprüche 1 bis 4, enthaltend Fructoseoligosaccharide und Mikroorganismen.

**6.** Tabletten gemäß Anspruch 5, **dadurch gekennzeichnet, dass** die Oligosaccharide aus Inulin bestehen.

**7.** Tabletten gemäß einem der Ansprüche 5 bis 6, **dadurch gekennzeichnet, dass** die Mikroorganismen aus Milchsäure produzierenden Bakterien bestehen.

**8.** Tabletten gemäß einem der Ansprüche 5 bis 7, **dadurch gekennzeichnet, dass** sie auch Polysaccharide wie mikrokristalline Cellulose und Stärke sowie weitere Additive wie Calciumdiphosphat enthalten.

## Revendications

**1.** Procédé de production de comprimés à forte teneur en matériel viable, par pressage d'une matière de base de comprimés qui contient des microorganismes vivants, **caractérisé en ce que** la matière de base de comprimés contient aussi des oligosaccharides à base de fructose et constitués de plus de deux motifs de monosaccharides.

**2.** Procédé conforme à la revendication 1, **caractérisé en ce que** les oligosaccharides se trouvent en une proportion pondérale de 40 à 99,5 % dans la matière de base de comprimés.

**3.** Procédé conforme à l'une des revendications 1 et 2, **caractérisé en ce que** les oligosaccharides sont de l'inuline.

**4.** Procédé conforme à l'une des revendications 1 à 3, **caractérisé en ce que** les microorganismes sont des bactéries produisant de l'acide lactique.

**5.** Comprimés produits conformément à l'une des revendications 1 à 4, qui contiennent des oligosaccharides à base de fructose et des microorganismes.

**6.** Comprimés conformes à la revendication 5, **caractérisés en ce que** les oligosaccharides sont de l'inuline.

**7.** Comprimés conformes à l'une des revendications 5 et 6, **caractérisés en ce que** les microorganismes sont des bactéries produisant de l'acide lactique.

**8.** Comprimés conformes à l'une des revendications 5 à 7, **caractérisés en ce qu'**ils contiennent en outre des polysaccharides comme de la cellulose microcristalline ou de l'amidon, ainsi que d'autres adjuvants comme du diphosphate de calcium.